# EUROPÄISCHE PATENTSCHRIFT

(11) **EP 0 491 328 B1**
(45) Veröffentlichungstag und Bekanntmachung des Hinweises auf die Patenterteilung: **27.03.1996**
(21) Anmeldenummer: 91121547.3
(22) Anmeldetag: 16.12.1991
(51) Int. Cl.: C07D 239/42

(54) **Verfahren zur Herstellung von Piperazinylpyrimidin-Derivaten**
Process for the preparation of piperazinyl-pyrimidin derivatives
Procédé de préparation de dérivés de pipérazinyl-pyrimidines

(30) Priorität: 18.12.1990 CH 4014/90
(43) Veröffentlichungstag der Anmeldung: 24.06.1992
(73) Patentinhaber: LONZA AG, CH-3945 Gampel/Wallis (CH)
(72) Erfinder: Kuo, David L., Dr., Brig (Kanton Wallis) (CH)
(74) Vertreter: Weinhold, Peter, Dr.

(56) Entgegenhaltungen:
- EP-A- 0 129 128

## Beschreibung

Die Erfindung betrifft ein neues Verfahren zur Herstellung von Piperazinylpyrimidin-Derivaten der allgemeinen Formel 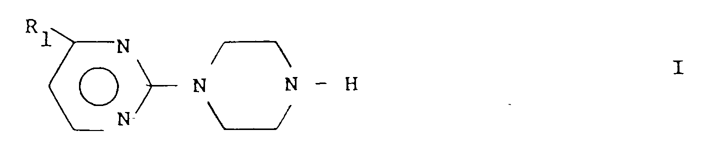
worin R₁ ein Wasserstoffatom oder eine C₁-C₄-Alkylgruppe, verzweigt oder unverzweigt, bedeutet.

Die Piperazinylpyrimidin-Derivate der allgemeinen Formel I sind wichtige Zwischenprodukte zur Herstellung von pharmazeutischen Produkten, wie beispielsweise zur Herstellung von 4-Methyl-2-piperazinylpyrimidin, welches den Blutzuckerspiegel senkt (EP-A 0 330 263).

Ein Verfahren zur Herstellung von Piperazinylpyrimidin-Derivaten ist in der EP-A 0 330 263 beschrieben. Bei diesem Verfahren wird ein 2-Chlorpyrimidin mit Piperazin in ein Piperazinylpyrimidin-Derivat überführt.
Ein Nachteil dieses Verfahrens liegt darin, dass Chlorpyrimidine schwer zugänglich sind und die Endprodukte in schlechter Ausbeute erhalten werden.

EP-A-0 129 128 beschreibt die Herstellung von Piperazinylpyrimidin-Derivaten durch Umsetzung von Piperazinderivaten mit methylierten Amidinsalzen. Eine Umsetzung mit ungesättigten Carbonylverbindungen ist nicht erwähnt.

Aufgabe der vorliegenden Erfindung war es, die Nachteile der bekannten Verfahren auszuschalten und ein einfaches und wirtschaftliches Verfahren zur Herstellung von Piperazinylpyrimidin-Derivaten zur Verfügung zu stellen, wobei das Endprodukt in guter Ausbeute und mit einem hohen Reinheitsgrad isoliert werden kann.

Diese Aufgabe wurde gemäss Patentanspruch 1 gelöst.

Das Verfahren zur Herstellung von Piperazinylpyrimidin-Derivaten der allgemeinen Formel 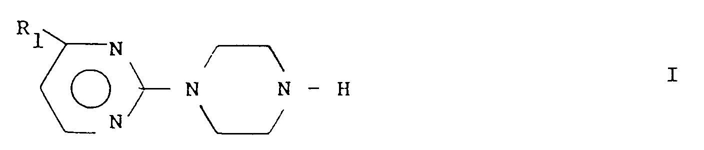
worin R₁ ein Wasserstoffatom oder eine C₁-C₄-Alkylgruppe, verzweigt oder unverzweigt, bedeutet, wird derart durchgeführt, dass man in der ersten Stufe Piperazin der Formel 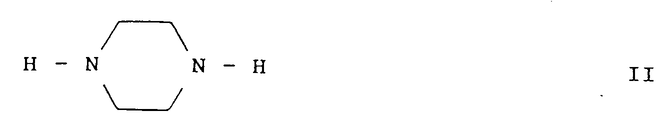
oder dessen Hydrat auf bekannte Weise sauer, d.h. in Anwesenheit einer Mineralsäure oder einer niederen aliphatischen Carbonsäure, mit Cyanamid, gemäss JP 51-39680, in ein Amidinsalz der Formel 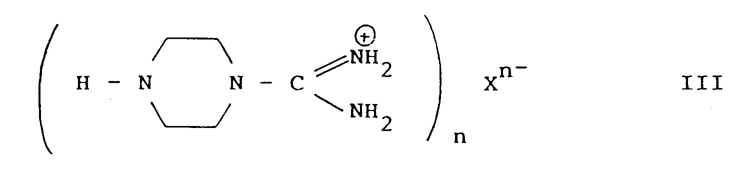
worin X ein Salzanion bedeutet und n der Wertigkeit dieses Salzanions entspricht, umsetzt, dieses gegebenenfalls isoliert und dann in der zweiten Stufe mit einer Carbonylverbindung der allgemeinen Formel 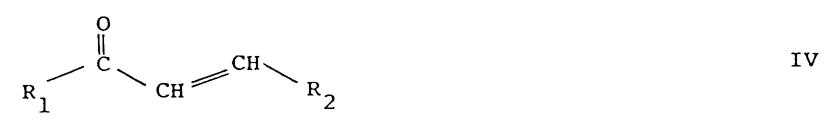
worin R₁ die genannte Bedeutung hat und R₂ eine C₁-C₄-Alkoxygruppe oder eine Aminogruppe, die substituiert ist, bedeutet, in Gegenwart einer Base zum Endprodukt umsetzt.

Zweckmässig wird die Umsetzung in der ersten Stufe, gemäss der JP 51-39680, mit 1 bis 1,5 mol Cyanamid, vorzugsweise mit 1,1 mol Cyanamid, bezogen auf 1 mol Piperazin oder dessen Hydrat, durchgeführt.

Zweckmässig wird die Umsetzung in der ersten Stufe bei einer Temperatur von 40 bis 60°C durchgeführt.

Zweckmässig wird die Umsetzung in der ersten Stufe in Gegenwart einer Mineralsäure durchgeführt. Als Mineralsäure können beispielsweise Schwefelsäure, Salzsäure oder Phosphorsäure angewendet werden. Dementsprechend bedeutet dann Xⁿ⁻ im Amidinsalz Chlorid, Sulfat oder Phosphat. Insbesondere wird als Mineralsäure konzentrierte Schwefelsäure angewendet.

Vorzugsweise werden die Mineralsäure und das Piperazin oder dessen Hydrat für die erste Stufe aequimolar eingesetzt. Als Lösungsmittel wird zweckmässig das selbe Lösungsmittel wie in der zweiten Stufe verwendet.

Nach einer üblichen Reaktionszeit von 1 bis 2 Stunden wird dann gegebenenfalls das Amidinsalz nach üblichen Aufarbeitungsmethoden, z.B. durch Einengen, erhalten.

Die zweite Stufe, die Umsetzung des Amidinsalzes mit einer Carbonylverbindung,erfolgt mit den Carbonylverbindungen gemäss der Formel 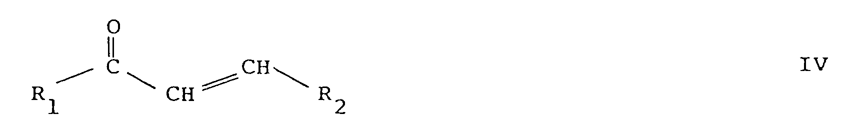
worin R₁ die genannte Bedeutung hat und R₂ eine C₁-C₄-Alkoxygruppe oder eine Aminogruppe, die substituiert ist, bedeutet.

Bevorzugte Verbindungen der Formel IV sind:
trans-4-Methoxy-3-buten-2-on (R₂ stellt eine Methoxygruppe dar)
N,N'-Dimethylaminoacrolein (R₂ stellt eine Dimethylaminogruppe dar)

Die Herstellung von N,N'-Dimethylaminoacrolein, als Vertreter der Verbindungen der allgemeinen Formel IV,erfolgt, ausgehend von Ethyl-vinylether,mit Dimethylformamid und POCl₃ in Dichlorethan (S.M. Makin et al., Zhurnal Organicheskoi Khimii, 1972, S.1394).

Zweckmässig wird die Umsetzung in der zweiten Stufe mit 0,5 bis 2 mol Carbonylverbindung, vorzugsweise mit 0,8 bis 1 mol, bezogen auf 1 mol Amidinsalz,durchgeführt, bei einer zweckmässigen Temperatur von 50°C bis zur Rückflusstemperatur des Lösungsmittels, vorzugsweise bei Rückflusstemperatur.

Die Umsetzung wird in der zweiten Stufe in einem polaren Lösungsmittel durchgeführt.
Als polare Lösungsmittel können niedrig siedende Alkohole dienen, wie beispielsweise Methanol oder Ethanol.
Dimethylsulfoxid oder Dimethylformamid können ebenfalls als Lösungsmittel geeignet sein.
Vorzugsweise wird als Lösungsmittel Methanol angewendet.

Die Umsetzung in der zweiten Stufe mit der Carbonylverbindung wird in Gegenwart einer Base durchgeführt.
Als Base kann ein Alkoholat angewendet werden, wie beispielsweise Natriummethylat oder Natriumethylat.
Vorzugsweise wird Natriummethylat angewendet.
Vorzugsweise wird die Base in einer Menge von 2 bis 5 mol, bezogen auf 1 mol der Carbonylverbindung, eingesetzt.

Nach einer üblichen Reaktionszeit von 3 bis 8 Stunden wird dann das Endprodukt nach üblichen Aufarbeitungsmethoden, wie z.B. durch Extraktion und Destillation,isoliert.

Vorzugsweise werden als Endprodukte 2-(1-Piperazinyl)pyrimidin und 4-Methyl-2-(1-piperazinyl)pyrimidin erhalten.

### Beispiel 1

### Herstellung von N,N'-Dimethylaminoacrolein (nicht erfindungsgemäss)

Bei 0°C wurde zu einer Lösung von 29,2 g Dimethylformamid (0,4 mole) in Dichlorethan (60 ml) tropfenweise eine Lösung von 30,6 g POCl₃ (0,2 mol) in Dichlorethan (30 ml) hinzugefügt.
Anschliessend wurde eine Lösung von Ethyl-vinylether (14,4 g, 0,2 mol) in Dichlorethan (20 ml) hinzugegeben.
Die Reaktionsmischung wurden 3 h lang auf 70°C erwärmt, dann auf 0°C abgekühlt,und anschliessend wurde H₂O (80 ml) hinzugegeben und das Ganze über Nacht gerührt. Bei 0°C wurde dann eine gesättigte K₂CO₃-Lösung (200 ml) tropfenweise hinzugefügt, dann wurde mit CHCl₃ 5 mal (je 50 ml) extrahiert, und die vereinigten organischen Phasen wurden 1 mal mit H₂O (100 ml) gewaschen und über Na₂SO₄ getrocknet.
Nach Abdestillieren des Lösungsmittels im Vakuum wurden 38,1 g einer hellorange-roten Flüssigkeit erhalten, die nach weiterer Destillation (0,8 mbar, 100°C) 11,9 g N,N'-Dimethylaminoacrolein, als gelbe Flüssigkeit, entsprechend einer Ausbeute von 60,3%, ergaben.

### Beispiel 2

### Herstellung von 2-(1-Piperazinyl)-amidinsulfat (nicht erfindungsgemäss)

Zu Piperazinhexahydrat (552 g, 2,78 mole) wurde innerhalb von 10 min 95,6%-ige Schwefelsäure (74,5 g, 0,72 mol) hinzugegeben. Diese Mischung wurde auf 50°C erwärmt, und dann wurde eine 25%-ige wässrige Lösung von Cyanamid (484 g, 2,88 mol), innerhalb von 2 h langsam hinzugefügt.
Nach weiterem Rühren für 2 h bei 50°C wurde innerhalb von 10 min 95,6%-ige Schwefelsäure (0,47 mole) hinzugefügt,was eine Temperaturerhöhung auf ca. 63°C zur Folge hatte. Dann wurde die Mischung bis zur Trockne eingeengt,und zu dieser klebrigen Masse (601 g) wurde abs. Methanol (800 ml) hinzugefügt und 1 h kühlgestellt.
Anschliessend wurde die Lösung filtriert, mit kaltem abs. Methanol 2 mal (100 ml) gewaschen.
Nach Trocknen im Vakuum wurden 328 g 2-(1-Piperazinyl)-amidinsulfat, entsprechend einer Ausbeute von 77,3%, erhalten. Die Mutterlauge wurde 2 Tage lang gekühlt, anschliessend filtriert, mit abs. Methanol 2 mal (50 ml) gewaschen und unter Vakuum getrocknet, wobei 43,3 g 2-(1-Piperazinyl)-amidinsulfat, entsprechend einer Ausbeute von 10,2%, erhalten wurden. Nach Vereinigung dieser beiden Fraktionen betrug die Gesamtausbeute 87,6%, bezogen auf das Ausgangsprodukt.

### Beispiel 3

### Verfahren zur Herstellung von 2-(1-Piperazinyl)pyrimidin

Zu 2-(1-Piperazinyl)-amidinsulfat (50 g, 0,14 mol) wurde abs. Methanol (100 ml) und anschliessend Natriummethylat, 30 % in Methanol, (52,9 g, 0,29 mol) hinzugegeben. Das Gemisch wurde bis zum Rückfluss erhitzt,und anschliessend wurde langsam N,N'-Dimethylaminoacrolein (12,3 g, 0,118 mol) innerhalb von 1,5 h hinzugegeben.
Anschliessend wurde die Mischung 4 h lang unter Rückfluss gerührt, dann auf Raumtemperatur abgekühlt, kaltes H₂O (300 ml) hinzugegeben und filtriert.
Das Filtrat wurde 4 mal mit CHCl₃ (100 ml) extrahiert und die vereinigten organischen Phasen wurden über Na₂SO₄ getrocknet. Nach Entfernen des Lösungsmittels im Vakuum wurden 21,4 g Produkt erhalten, und nach Destillation (0,45 mbar, 98-100°C) wurden dann 16,1 g Produkt, entsprechend einer Ausbeute von 83%, bezogen auf das Amidinsulfat, mit einem Gehalt von mehr als 96% (GC) erhalten.

### Beispiel 4

### Herstellung von 4-Methyl-2-(1-piperazinyl)pyrimidin

Zu einer Lösung von 2-(1-Piperazinyl)amidinsulfat (13,6 g, 38,5 mmol) in abs. Methanol (20 ml) wurde Natriummethanolat (24,2 ml, 128 mmol) hinzugegeben.
Anschliessend wurde die Mischung 10 min lang auf 80°C am Rückfluss erwärmt.
Dann wurde trans-4-Methoxy-3-buten-2-on (5,14 g, 51,3 mmol), gelöst in absolutem Methanol (20 ml), innerhalb von 2,5 h hinzugegeben.
Nach beendeter Reaktion wurde die Mischung noch 2 h lang bei 80°C gerührt. Nach dem Abkühlen wurde 5 mal mit CHCl₃ (20 ml) extrahiert, und die vereinigten organischen Phasen wurden 1 mal mit H₂O (20 ml) gewaschen und über Na₂SO₄ getrocknet.
Nach Entfernen des Lösungsmittels im Vakuum wurden 6,14 g hellbrauner Rückstand erhalten.
Nach Destillation (130-150°C, 0,5 mbar) wurden 4,93 g 4-Methyl--2-(1-piperazinyl)pyrimidin, entsprechend einer Ausbeute von 53,9%, bezogen auf eingesetztes Amidinsulfat, als farbloses Oel erhalten.

## Patentansprüche

1. Verfahren zur Herstellung von Piperazinylpyrimidin-Derivaten der allgemeinen Formel 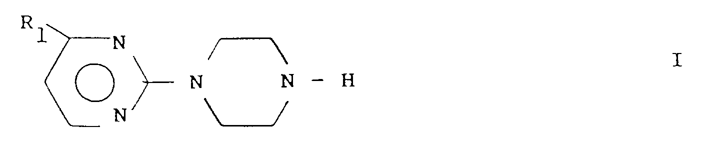 worin R₁ ein Wasserstoffatom oder eine C₁-C₄-Alkylgruppe, verzweigt oder unverzweigt, bedeutet, dadurch gekennzeichnet, dass man in der ersten Stufe Piperazin der Formel 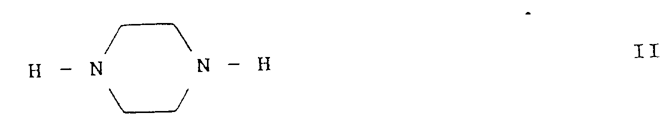 oder dessen Hydrat in Anwesenheit einer Mineralsäure oder einer niederen aliphatischen Carbonsäure mit Cyanamid in ein Amidinsalz der Formel 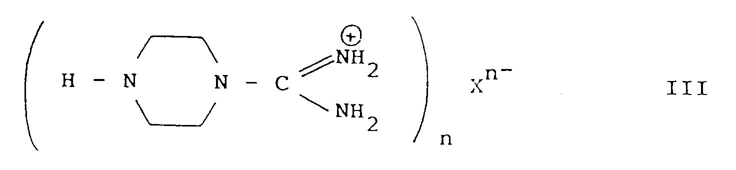 worin X ein Salzanion bedeutet und n der Wertigkeit dieses Salzanions entspricht,umsetzt, dieses gegebenenfalls isoliert und dann in der zweiten Stufe mit einer Carbonylverbindung der allgemeinen Formel 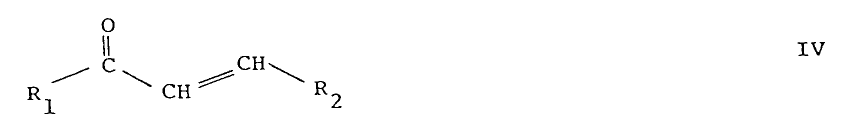 worin R₁ die genannte Bedeutung hat und R₂ eine C₁-C₄-Alkoxygruppe oder eine Aminogruppe, die substituiert ist, bedeutet, in Gegenwart einer Base in einem polaren Lösungsmittel zum Endprodukt umsetzt.

2. Verfahren nach Patentanspruch 1, dadurch gekennzeichnet, dass man die Umsetzung in der ersten Stufe mit 1 bis 1,5 mol Cyanamid, bezogen auf 1 mol Piperazin oder dessen Hydrat, durchführt.

3. Verfahren nach mindestens einem der Patentanprüche 1 und 2, dadurch gekennzeichnet, dass man die Umsetzung in der ersten Stufe in Gegenwart einer Mineralsäure durchführt.

4. Verfahren nach mindestens einem der Patentansprüche 1 bis 3, dadurch gekennzeichnet, dass man die Umsetzung in der ersten Stufe bei einer Temperatur von 40 bis 60°C durchführt.

5. Verfahren nach mindestens einem der Patentansprüche 1 bis 4, dadurch gekennzeichnet, dass man als Carbonylverbindung in der zweiten Stufe trans-4-Methoxy-3-buten-2-on einsetzt.

6. Verfahren nach mindestens einem der Patentansprüche 1 bis 4, dadurch gekennzeichnet, daß man als Carbonylverbindung in der zweiten Stufe N,N'-Dimethylaminoacrolein einsetzt.

7. Verfahren nach mindestens einem der Patentansprüche 1 bis 6, dadurch gekennzeichnet, daß man die Umsetzung in der zweiten Stufe bei einer Temperatur von 50°C bis zur Rückflußtemperatur des Lösungsmittels durchführt.

8. Verfahren nach mindestens einem der Patentansprüche 1 bis 7, dadurch gekennzeichnet, daß man die Umsetzung in der zweiten Stufe in Gegenwart eines Alkoholats als Base durchführt.

9. Verfahren zur Herstellung von Piperazinylpyrimidin-Derivaten der allgemeinen Formel 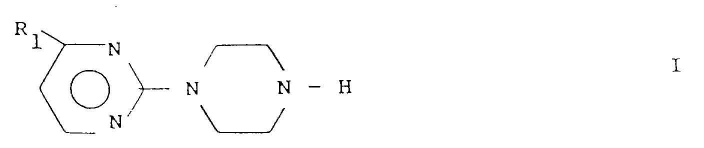 worin R₁ ein Wasserstoffatom oder eine C₁-C₄-Alkylgruppe, verzweigt oder unverzweigt, bedeutet, dadurch gekennzeichnet, daß man ein Amidinsalz der Formel 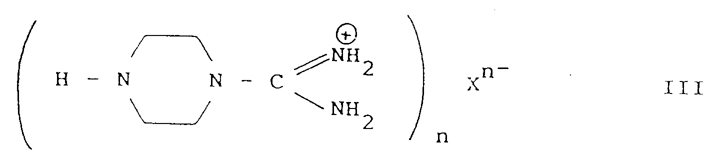 worin X ein Salzanion bedeutet und n der Wertigkeit dieses Salzanions entspricht, mit einer Carbonylverbindung der allgemeinen Formel 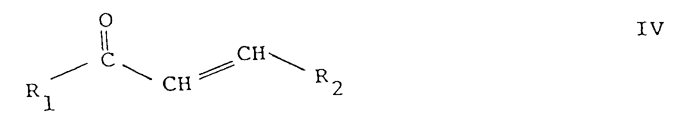 worin R₁ die genannte Bedeutung hat und R₂ eine C₁-C₄-Alkoxygruppe oder eine Aminogruppe, die substituiert ist, bedeutet, in Gegenwart einer Base in einem polaren Lösungsmittel zum Endprodukt umsetzt.

10. Verfahren nach Patentanspruch 9, dadurch gekennzeichnet, daß man als Carbonylverbindung trans-4-Methoxy-3-buten-2-on oder N,N'-Dimethylaminoacrolein einsetzt.

11. Verfahren nach einem der Patentansprüche 9 oder 10, dadurch gekennzeichnet, daß man die Umsetzung bei einer Temperatur von 50°C bis zur Rückflußtemperatur des Lösungsmittels durchführt.

12. Verfahren nach einem der Patentansprüche 9 bis 11, dadurch gekennzeichnet, daß man die Umsetzung in Gegenwart eines Alkoholats als Base durchführt.

## Claims

1. A process for the production of piperazinylpyrimidine derivatives of the general formula: 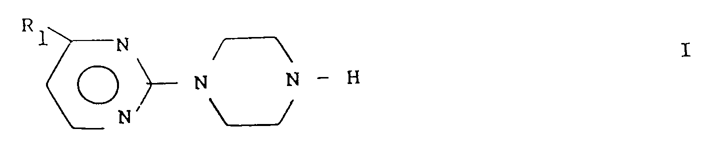 wherein R₁ is a hydrogen atom or a C₁-C₄ alkyl group, branched or unbranched, characterized in that, in a first stage, piperazine of the formula: 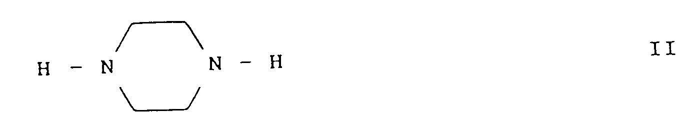 or its hydrate is reacted with cyanamide in the presence of a mineral acid or a lower aliphatic carboxylic acid to an amidine salt of the formula: 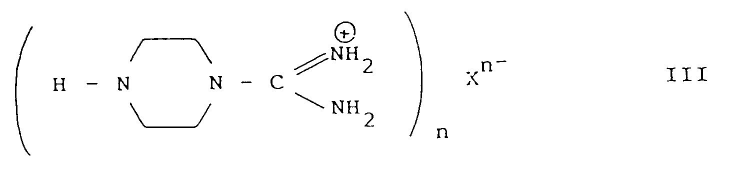 wherein X is a salt anion and n corresponds to the valence of said salt anion, the amidine salt being optionally isolated, and then, in a second stage, the amidine salt is reacted, in a polar solvent in the presence of a base, with a carbonyl compound of the general formula: 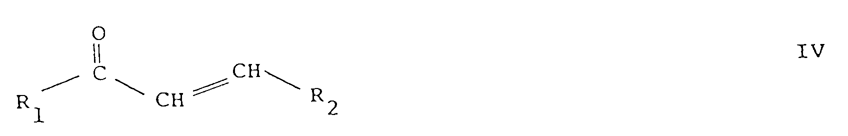 wherein R₁ has the above meaning and R₂ is a C₁-C₄ alkoxy group or an amino group which is substituted, to the end product.

2. The process according to claim 1, characterized in that the reaction of the first stage is performed with 1 to 1.5 moles of cyanamide, relative to 1 mole of piperazine or its hydrate.

3. The process according to at least one of the claims 1 and 2, characterized in that the reaction of the first stage is performed in the presence of a mineral acid.

4. The process according to at least one of the claims 1 to 3, characterized in that the reaction of the first stage is performed at a temperature of 40 - 60 °C.

5. The process according to at least one of the claims 1 to 4, characterized in that, in the second stage, trans-4-methoxy-3-buten-2-one is used as the carbonyl compound.

6. The process according to at least one of the claims 1 to 4, characterized in that, in the second stage, N,N'-dimethylaminoacrolein is used as the carbonyl compound.

7. The process according to at least one of the claims 1 to 6, characterized in that the reaction of the second stage is performed at a temperature of from 50 °C to the reflux temperature of the solvent.

8. The process according to at least one of the claims 1 to 7, characterized in that the reaction of the second stage is performed in the presence of an alcoholate as the base.

9. A process for the production of piperazinylpyrimidine derivatives of the general formula: 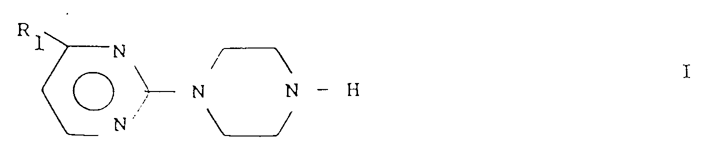 wherein R₁ is a hydrogen atom or a C₁-C₄ alkyl group, branched or unbranched, characterized in that an amidine salt of the formula: 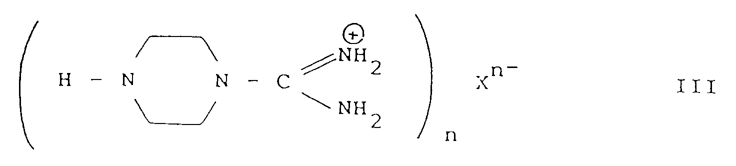 wherein X is a salt anion and n corresponds to the valence of said salt anion, is reacted, in a polar solvent in the presence of a base, with a carbonyl compound of the general formula: 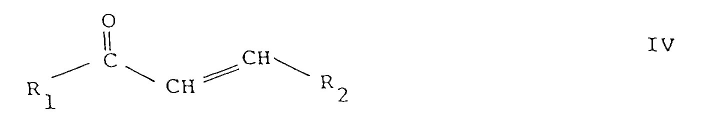 wherein R₁ has the above meaning and R₂ is a C₁-C₄ alkoxy group or an amino group which is substituted, to the end product.

10. The process according to claim 9, characterized in that trans-4-methoxy-3-buten-2-one or N,N'-dimethylaminoacrolein is used as the carbonyl compound.

11. The process according to one of the claims 9 or 10, characterized in that the reaction is performed at a temperature of from 50 °C to the reflux temperature of the solvent.

12. The process according to one of the claims 9 to 11, characterized in that the reaction is performed in the presence of an alcoholate as the base.

## Revendications

1. Procédé pour la préparation de dérivés de pipérazinylpyrimidine de la formule générale 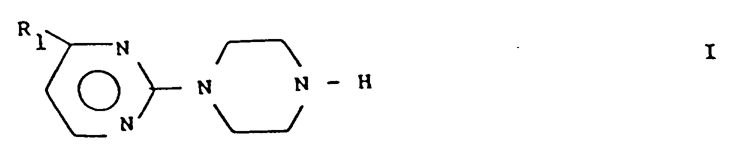 dans laquelle R₁ signifie un atome d'hydrogène ou un groupe alkyle C₁-C₄, ramifié ou non ramifié, caractérisé en ce que l'on met en réaction dans la première étape la pipérazine de la formule 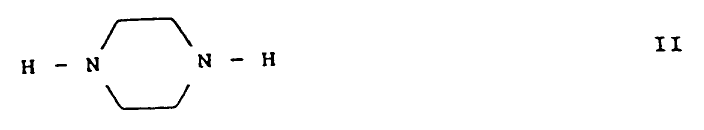 ou son hydrate en présence d'un acide minéral ou d'un acide carboxylique aliphatique inférieur avec le cyanamide dans un sel amidine de la formule 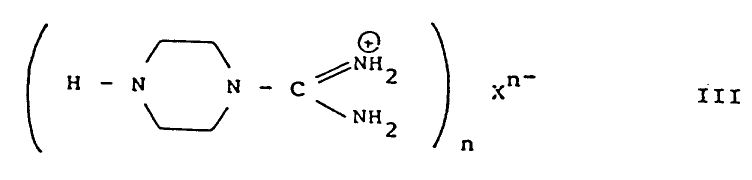 dans laquelle X signifie un anion sel et n correspond à la valence de cet anion sel, on isole éventuellement celui-ci et ensuite dans la deuxième étape, on met en réaction avec un composé carbonyle de la formule générale 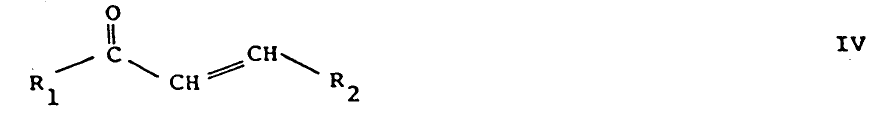 dans laquelle R₁ a la signification précitée et R₂ signifie un groupe alcoxy C₁-C₄ ou un groupe amino, substitué, en présence d'une base dans un solvant polaire pour obtenir le produit final.

2. Procédé selon la revendication 1, caractérisé en ce que l'on conduit la réaction dans la première étape avec 1 jusqu'à 1,5 moles de cyanamide rapportées à 1 mole de pipérazine ou de son hydrate.

3. Procédé selon au moins l'une des revendications 1 et 2, caractérisé en ce que l'on conduit la réaction dans la première étape en présence d'un acide minéral.

4. Procédé selon au moins l'une des revendications 1 à 3, caractérisé en ce que l'on conduit la réaction dans la première étape à une température de 40 à 60°C.

5. Procédé selon au moins l'une des revendications 1 à 4, caractérisé en ce que l'on met en oeuvre la trans-4-méthoxy-3-butèn-2-one comme composé carbonyle dans la première étape.

6. Procédé selon au moins l'une des revendications 1 à 4, caractérisé en ce que l'on met en oeuvre la N,N'-diméthylamino-acroléine comme composé carbonyle dans la seconde étape.

7. Procédé selon au moins l'une des revendications 1 à 6, caractérisé en ce que l'on conduit la réaction dans la seconde étape à une température de 50°C jusqu'à la température de reflux du solvant.

8. Procédé selon au moins l'une des revendications 1 à 7, caractérisé en ce que l'on conduit la réaction dans la seconde étape en présence d'un alcoolat comme base.

9. Procédé pour la préparation de dérivés de la pipérazinylpyrimidine de la formule générale 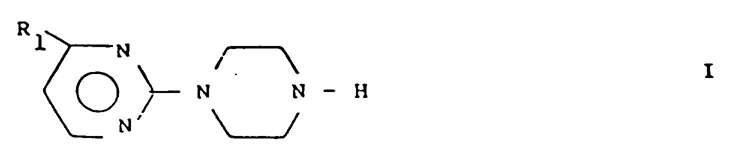 dans laquelle R₁ signifie un atome d'hydrogène ou un groupe alkyle C₁-C₄, ramifié ou non ramifié, caractérisé en ce que l'on met en réaction un sel amidine de la formule 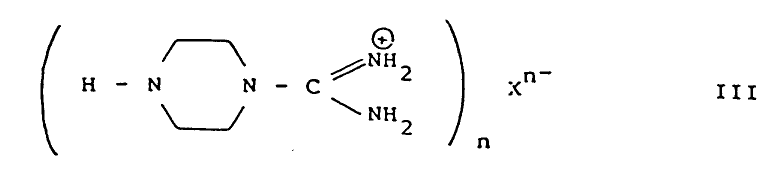 dans laquelle X signifie un anion sel et n correspond à la valence de cet anion sel, avec un composé carbonyle de la formule générale 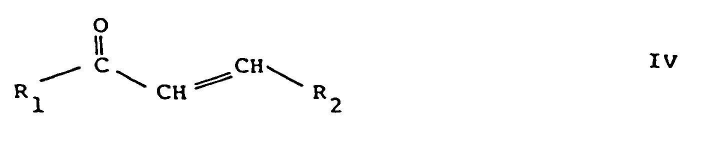 dans laquelle R₁ a la signification précitée et R₂ signifie un groupe alcoxy C₁-C₄ ou un groupe amino, qui est substitué, en présence d'une base dans un solvant polaire pour obtenir le produit final.

10. Procédé selon la revendication 9, caractérisé en ce que l'on met en oeuvre la trans-4-méthoxy-3-butèn-2-one ou la N,N'-diméthylaminoacroléine comme composé carbonyle.

11. Procédé selon l'une des revendications 9 ou 10, caractérisé en ce que l'on conduit la réaction à une température de 50°C jusqu'à la température de reflux du solvant.

12. Procédé selon l'une des revendications 9 à 11, caractérisé en ce que l'on conduit la réaction en présence d'un alcoolat comme base.
